# EUROPÄISCHE PATENTSCHRIFT

(11) **EP 2 114 174 B1**
(45) Veröffentlichungstag und Bekanntmachung des Hinweises auf die Patenterteilung: **08.07.2015**
(21) Anmeldenummer: 08707214.6
(22) Anmeldetag: 23.01.2008
(51) Int. Cl.: A23L 1/30, A23L 2/52, A61K 31/14, A61K 31/155, A61P 3/00, A61P 19/00, A61P 25/00, A61K 31/205

(54) **VERWENDUNG VON GUANIDINOESSIGSÄURE(-SALZEN) IN KOMBINATION MIT BETAIN UND/ODER CHOLIN ZUR HERSTELLUNG EINES GESUNDHEITSFÖRDERNDEN MITTELS**
USE OF GUANIDINOACETIC ACID (SALTS) COMBINED WITH BETAINE AND/OR CHOLINE FOR PRODUCING A HEALTH-PROMOTING AGENT
UTILISATION (DE SELS) D'ACIDE GUANIDINOACÉTIQUE EN ASSOCIATION AVEC DE LA BÉTAÏNE ET/OU DE LA CHOLINE POUR PRODUIRE UN AGENT BÉNÉFIQUE POUR LA SANTÉ

(30) Priorität: 31.01.2007 DE 102007004781
(43) Veröffentlichungstag der Anmeldung: 11.11.2009
(73) Patentinhaber: AlzChem AG, 83308 Trostberg (DE)
(72) Erfinder: GASTNER, Thomas, 84518 Garching an der Alz (DE); KRIMMER, Hans-Peter, 84558 Kirchweidach (DE)
(74) Vertreter: Weickmann & Weickmann
(86) Internationale Anmeldenummer: PCT/EP2008/000501
(87) Internationale Veröffentlichungsnummer: WO 2008/092591

(56) Entgegenhaltungen:
- WO-A-2004/000042
- WO-A1-2007/014756
- DE-A1-102005 009 990
- DE-A1-102006 035 801
- US-A1- 2005 085 543
- BORSOOK H ET AL: "THE BIOCHEMICAL BASIS OF BETAINE-GLYCOCYAMINE THERAPY" ANNALS OF WESTERN MEDICINE AND SURGERY, LOS ANGELES COUNTY MEDICAL ASSOCIATION, LOS ANGELES, US, Bd. 5, Nr. 10, Oktober 1951 (1951-10), Seiten 825-829, XP009075328

## Beschreibung

Die vorliegende Erfindung betrifft die Verwendung von Guanidinoessigsäure bzw. deren Salze zur Herstellung eines gesundheitsfördernden Mittels.

Guanidinoessigsäure wurde erstmals von C. J. Weber im Jahre 1934 aus dem Urin von Hunden und Menschen isoliert. Bereits Weber vermutete, dass es sich um den metabolischen Vorläufer des Kreatins handelt (Weber, C. J., Proc. Sot. Exp. Biol. and Med., 33, 172 (1934)).

Wenig später konnte gezeigt werden, dass Guanidinoessigsäure tatsächlich eine bei Tieren und auch im Menschen vorkommende körpereigene Substanz ist, welche bei der Biosynthese des Kreatins eine zentrale Rolle einnimmt. Kreatin kann sowohl durch die Nahrung aufgenommen als auch endogen gebildet werden. Die Biosynthese geht von Glycin und L-Arginin aus. Bei Säugetieren wird vor allem in den Nieren, aber auch in der Leber und der Bauchspeicheldrüse, durch das Enzym Aminotransferase die Guanidino-Gruppe des L-Arginins gespalten und eine N-C-N-Gruppe auf das Glycin übertragen. Das L-Arginin wird hierbei in L-Ornithin umgewandelt. Die so gebildete Guanidinoessigsäure wird im nächsten Schritt, bei Vertebraten geschieht dies ausschließlich in der Leber, mit Hilfe des Enzyms Transmethylase in Kreatin umgewandelt. Hierbei dient das S-Adenosylmethionin als Methylgruppen-Donor. Das Kreatin wird anschließend über den Blutkreislauf zu den Zielorganen transportiert. Der Transport durch die Zellmembran in die Zellen geschieht hierbei durch einen spezifischen NaCl-abhängigen Kreatin-Transporter (Speer O, Neukomm LJ, Murphy RM, Zanolla E, Schlattner U, Henry H, Snow RJ, Wallimann T. Creatine transporters: a reappraisal. Mol Cell Biochem. 2004 Jan-Feb;256-257(1-2):407-24).

Kreatin nimmt im Energiestoffwechsel der Zelle eine wichtige Rolle ein, wobei es als energiereiches Phosphokreatin neben dem Adenosintriphosphat (ATP) eine wesentliche Energiereserve des Muskels darstellt. Im Ruhezustand des Muskels kann ATP auf Kreatin eine PhosphatGruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion durch das Enzym Kreatinkinase eine Phosphatgruppe auf Adenosindiphosphat übertragen und somit ATP zurückbilden. Dies wird auch als Lohmann-Reaktion bezeichnet.

Weiterhin besitzt Kreatin eine wichtige Funktion bei der Übertragung von Energie in der Zelle. Das so genannte Kreatin-Shuttle-System transportiert Energie von den Mitochondrien an die Stellen in der Zelle, an denen die Energie benötigt wird.

Bei starker und über längere Zeit anhaltender Muskelarbeit sind die natürlich im Körper vorhandenen Kreatin-Vorräte rasch erschöpft. Aus diesem Grund haben sich insbesondere bei Leistungssportlern gezielte Kreatin-Gaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper über die Nieren ausgeschieden wird. Weiterhin wandelt sich Kreatin mit einer konstanten Rate in das zyklische Abbauprodukt Kreatinin um, welches ebenfalls über die Nieren ausgeschieden wird und somit einen zweiten metabolischen Abbauweg darstellt.

Seit Ende der 70er Jahre des vergangenen Jahrhunderts wird die ergogene Wirkung von Kreatin systematisch untersucht. Bis heute wurden über 300 Studien im Sportbereich durchgeführt, wobei etwa 80 % dieser Studien signifikant positive Effekte des Kreatins auf die Muskelmasse, die Muskelkraft, die fettfreie Körpermasse und die Leistung bei maximaler, kurzzeitiger Muskelanstrengung in verschiedenen Sportarten zeigte. Kreatin-Monohydrat ist heute das wichtigste Nahrungsergänzungsmittel im Sportbereich.
Erst vor kurzer Zeit sind weitere interessante Eigenschaften des Kreatins bekannt geworden. So wurden in zwei Studien signifikant positive Wirkungen einer oralen Kreatin-Supplementation auf die Gehirnleistung und die Konzentrationsfähigkeit nachgewiesen (Rae, Caroline et al.: Oral creatine monohydrate supplementation improves brain performance: a double-blind, placebo-controlled, cross-over trial. Proceedings of the Royal Society of London, Series B: Biological Sciences (2003), 270(1529), 2147-2150; Watanabe, Airi et al.: Effects of creatine on mental fatigue and cerebral hemoglobin oxygenation. Neuroscience Research (Oxford, United Kingdom) (2002), 42(4), 279-285).

Weiterhin konnte gezeigt werden, dass Kreatin antioxidative und neuroprotektive Eigenschaften besitzt und somit auch zur Vorbeugung von Schädigungen der Zellen durch Umwelteinflüsse eingesetzt werden kann (Sestili, Piero et al.: Creatine supplementation affords cytoprotection in oxidatively injured cultured mammalian cells via direct antioxidant activity. Free Radical Biology & Medicine (2006), 40(5), 837-849; P. Klivenyi et al.: Neuroprotective effects of creatine in a transgenic animal model of amyotrophic lateral sclerosis. Nature Medicine 5, 347-350 (1999)).
Als einfach zu messender Indikator für oxidativen Stress im menschlichen Körper kann das Verhältnis von Cystein zu Cystin im Plasma dienen (Hack et al.: BLOOD 92 (1998) 59-67). Das Verhältnis dieser Komponenten spiegelt hierbei direkt den Redoxzustand wieder, wobei oxidativer Stress durch die Erhöhung des Cystin-Wertes gekennzeichnet ist. Es ist bekannt, dass oxidativer Stress im menschlichen Körper durch eine Supplementierung von Kreatin vermieden werden kann. Die Cystinwerte ließen sich auch bei älteren Probanden durch eine Supplementierung mit wenigen Gramm Kreatin pro Tag deutlich senken und führten zu Werten, wie sie bei gesunden jungen Menschen zu finden sind (US 6,927,231). Kreatin führt zu einer deutlichen Verminderung von oxidativem Stress und wirkt somit präventiv gegen degenerative Alterungsprozesse. Kreatin und seine Derivate werden deshalb in Zukunft auch im Anti-Aging Bereich stark an Bedeutung gewinnen.

Die positiven Effekte von Kreatin werden derzeit auch im medizinischen Bereich intensiv untersucht, wobei sich Kreatin bei der Behandlung von Parkinson und der Amyotrophen Lateralen Sklerose (ALS) in der klinischen Phase 3 und bei Corea-Huntington in Phase 2 befindet (EP 804 183 B1). Auch von einem erfolgreichen Einsatz von Kreatin als Therapeutikum gegen Asthma wurde bereits berichtet (EP 911 026 B1). Beim Aufbau von Knochen zeigte Kreatin sowohl in vitro als auch in vivo positive Effekte. Der Einsatz zur Stärkung der Knochen und zur Behandlung und Vorbeugung von degenerativen Knochen- und Knorpel-Erkrankungen wie etwa Osteoporose wurde untersucht und lieferte sehr positive Ergebnisse (EP 1 100 488 B1; Gerber, I et al.: Stimulatory effects of creatine on metabolic activity, differentiation and mineralization of primary osteoblast-like cells in monolayer and micromass cell cultures. European Cells and Materials (2005), 10, 8-22; Chilibeck, P. D. et al.: Creatine monohydrate and resistance training increase bone mineral content and density in older men. Journal of Nutrition, Health & Aging (2005), 9(5), 352-355).

Weiterhin ist bekannt, dass eine Kreatin-Supplementierung zu einer Erhöhung der Körpermasse führt. Dies ist zu Anfang auf eine vermehrte Aufnahme von Wasser in den Muskel zurückzuführen. Langfristig gesehen führt Kreatin aber indirekt durch vermehrte Proteinsynthese oder einen verminderten Proteinkatabolismus in den Myofibrillen zu einer Erhöhung der Muskelmasse (Int J Sports Med 21 (2000), 139-145). Als Ergebnis erhält man somit eine erhöhte fettfreie Körpermasse.

US 2005/085543 A1 beschreibt die Verwendung von Kreatinverbindungen für die Heilung von Knochen- oder Knorpeldefekten.

Neben dem Kreatin selbst, also dem Kreatin-Monohydrat, haben sich zwischenzeitlich aber auch zahlreiche Kreatin-Salze, wie das Kreatinascorbat, -citrat, -pyruvat und andere, ebenfalls als geeignete Nahrungsergänzungsmittel erwiesen. Stellvertretend seien an dieser Stelle das europäische Patent EP 894 083 und die deutsche Offenlegungsschrift DE 197 07 694 A1 als Stand der Technik genannt.

Mehrere Arbeitsgruppen konnten bereits in den 50er Jahren des letzten Jahrhunderts in klinischen Studien zeigen, dass die Verabreichung von Guanidinoessigsäure in Kombination mit Betain bei Herzkrankheiten einen positiven Einfluss auf den Krankheitsverlauf hat. Die Patienten berichteten von einer deutlichen Verbesserung ihres Allgemeinbefindens. Weiterhin wurden eine verbesserte Ausdauer bei körperlicher Belastung und eine erhöhte Muskelkraft schon nach kurzer Behandlungsdauer festgestellt. Auch berichteten die Patienten von einer verbesserten Libido. 200 Patienten wurde eine Dosis von 30mg/kg täglich über ein Jahr verabreicht. Nebenwirkungen konnten nicht beobachtet werden (Borsook H.; Borsook M.E.: The biochemical basis of betaine-glycocyamine therapy. In: Annals of western medicine and surgery 5(10), 825, 1951).

Die internationale Patentanmeldung WO 91/07 954 offenbart die Verwendung von Guanidinoessigsäure in Kombination mit Methionin oder S-Adenosylmethionin zur Erhöhung des Kreatinspiegels im Muskel. Als Einsatzbereich werden Zustände genannt, welche einen höheren Kreatinspiegel im Muskel erfordern. Es werden hierbei sowohl medizinische Anwendungen als auch der Bereich der Sporternährung beansprucht. Hierbei wird die Behauptung aufgestellt, das Kreatin zur Erhöhung des Kreatinspiegels unwirksam ist. Diese Behauptung konnte inzwischen durch zahlreiche Arbeiten wiederlegt werden (z. B. Persky, A. M., Brazeau, G. A.: Clinical Pharmacology of the Dietary Supplement Creatine Monohydrate. In: Pharmacol Rev, 2001, 53, 161-176).

Die internationale Patentanmeldung WO 2004/000 297 beschreibt eine Mischung für die Ernährung oder für pharmazeutische Zwecke, welche für Säugetieren eingesetzt wird. Diese besteht aus einer Proteinfraktion, welche L-Serin enthält, und als weitere Komponente Guanidinoessigsäure. Die Mischung muss hierbei frei von Glycin sein oder nach der Hydrolyse der Mischung ein Verhältnis von L-Serin zu Glycin von größer als 2,7 zu 1 enthalten. Als mögliche Produktform werden Lösungen, Emulsionen, Suspensionen, Gele, Riegel, Süßigkeiten und vorzugsweise Pulver vorbeschrieben.

Dieses Verhältnis von L-Serin zu Glycin von größer 2,7 zu 1 ist in Nahrungsmitteln und Tierfutter normal nicht anzutreffen. Tierische Rohstoffe wie z.B. Tiermehl enthalten deutlich mehr Glycin als Serin (Amino acids of meals of animal origin. de Vuyst, A. Univ. Louvain, Belgum, Agricultura (Heverlee, Belgium) (1964), 12(1), 141-51). In pflanzlichen Rohstoffen ist das Verhältnis zwischen Glycin und Serin vorwiegend ausgeglichen.

WO 2004/000042 A offenbart Verfahren und Zusammensetzungen für die Behandlung oder Prävention von Katabolismus oder zur Stimulierung von Anabolismus in Säugetieren, die metabolischem Stress ausgesetzt sind. In diesem Zusammenhang werden Methyldonoren und ggf. Methylakzeptoren verabreicht.

DE 10 2005 009990 A1 betrifft Salze, Anlagerungs- und Komplexverbindungen der Guanidinoessigsäure, u.a. mit Betain oder Cholin, die beispielsweise als Nahrungsergänzungsmittel, als Futtermittel und in kosmetischen oder dermatologischen Zubereitungen verwendet werden können.

WO 2007/014756 A1 beschreibt eine Flüssigformulierung auf Basis einer Guandinoessigsäurekomponente für die menschliche und tierische Ernährung. Die beschriebene Zusammensetzung beinhaltet eine wässrige Lösung einer Guandinoessigsäurekomponente und einen Methylgruppendonor der Reihe Cholin, Methionin und Betain.

Die Aufnahme von Kreatin in die Muskulatur wird von einem NaCl-abhängigen Kreatin-Transporter gesteuert und kann durch die gleichzeitige Aufnahme einer großen Menge an Kohlenhydraten oder Proteinen positiv beeinflusst werden. Dabei zeigte sich, dass die Kombination von Kreatin und Kohlenhydraten zu einem um 60 % höheren Anstieg der Kreatin-Gehalte im Muskel im Vergleich zur alleinigen Einnahme von Kreatin führen kann (Green AL, Hultman E, Macdonald IA, Sewell DA, Greenhaff PL. Carbohydrate ingestion augments skeletal muscle creatine accumulation during creatine supplementation in humans. Am J Physiol. 1996 Nov;271 (5 Pt 1):E821-6).

Neben seinen unbestrittenen positiven physiologischen Eigenschaften besitzt Kreatin aber auch den Nachteil, dass es in den entsprechenden wässrigen Lösungen keine ausgeprägte Stabilität besitzt. Kreatin zyklisiert hierbei durch die Abspaltung von Wasser zum Kreatinin. Die Zyklisierungsgeschwindigkeit ist vom pH-Wert der Lösung und der Temperatur abhängig, wobei die Konzentration keine Rolle spielt. Besonders im neutralen und sauren pH-Bereich verläuft die Umwandlung in Kreatinin sehr schnell. Aufgrund des schnellen Abbaus von Kreatin in diesem Milieu ist der Einsatz in wässrigen oder feuchten Formulierungen für die menschliche und tierische Ernährung praktisch ausgeschlossen. Bereits der pH-Wert des Magens von 1 bis 2 kann je nach Verweilzeit zu einem deutlichen Abbau des Kreatins zu Kreatinin führen. (Greenhaff, P.L.: Factors Modifying Creatine Accumulation in Human Skeletal Muscle. In: Creatine. From Basic Science to Clinical Application. Medical Science Symposia Series Volume 14, 2000, 75-82).

Aus den geschilderten Nachteilen des Standes der Technik hat sich für die vorliegende Erfindung die Aufgabe gestellt, ein gesundheitsförderndes Mittel bereitzustellen, welches die Körperzellen effizienter mit Kreatin versorgt und somit die bekannten positiven physiologischen Wirkungen einer Supplementation mit Kreatins im menschlichen und tierischen Körper in noch höherem Maße zu erzielt. Die negativen Eigenschaften des Kreatins, wie etwa dessen geringe Stabilität im wässrigen und sauren Millieu, sollten umgangen werden. Hierbei sind vor allem auch die geringe Stabilität von Kreatin in Nahrungszubereitungen mit einem höheren Wassergehalt zu nennen, was die Einsatzmöglichkeiten des Kreatins in industriell hergestellten Nahrungsmitteln deutlich einschränkt. Weiterhin stellt die Instabilität nach der Aufnahme im Magen ein großes Problem dar. Ziel war es somit, den Körper effizienter mit Kreatin zu versorgen als dies durch eine direkte Gabe von Kreatin geschehen kann.

Um die Kreatingehalte in den Körperzellen optimal zu steigern, war bisher die gleichzeitige Aufnahme einer großen Menge an Kohlenhydraten oder Proteinen notwendig. Auch dies sollte möglichst vermieden werden, da hierdurch große Mengen an Insulin ausgeschüttet werden, was langfristig zu gesundheitlichen Problemen führen kann.

Gelöst wurde diese Aufgabe durch die Verwendung von Guandinoessigsäure und/oder deren Salzen in Kombination mit Cholin und/oder Betain zur Herstellung eines Mittels zur Verbesserung der Gehirnleistung, zur Verbesserung des Knochenwachstums und der Mineralisierung der Knochen, zur Verbesserung des Knorpelwachstums bei Menschen oder Wirbeltieren, wobei das molare Verhältnis der eingesetzten Menge an Guandinoessigsäure zu Cholin und/oder Betain im Bereich von 1:1 liegt.

Die Verwendung von Guanidinoessigsäure und deren Salzen in Kombination mit Cholin und/oder
Betain zur Herstellung eines Mittels, zur Milderung von Alterungsprozessen, als antioxidativer und neuroprotektiver Wirkstoff, zur Senkung des Cholesterin- und Triglyceridwertes, zur Vorbeugung von Entzündungsprozessen und zur Senkung des Blutzuckerspiegels bei Menschen oder Wirbeltieren wird hierin ebenfalls beschrieben.

Es konnte gezeigt werden, dass durch die Verwendung von Guanidinoessigsäure in Kombination mit Cholin und/oder Betain die Aufgabenstellung voll erfüllt werden konnte, nämlich die Körperzellen optimal mit Kreatin zu versorgen. Hierbei wurden bei Menschen und Wirbeltieren die vom Kreatin bekannten positiven Effekte mit noch höherer Wirksamkeit erzielt. Überraschend hat sich herausgestellt, dass die neuen Mittel eine deutlich höhere Bioverfügbarkeit aufweisen und somit besser in die Zellen aufgenommen werden, als dies bisher bei der Verwendung von Kreatin bekannt war.

Im Gegensatz zu Kreatin bzw. Kreatin-Monohydrat weisen Guanidinoessigsäure und deren Salze auch in saurer Lösung, wie sie im Magen auftritt, eine deutlich höhere Stabilität auf. Überraschenderweise hat sich als besonders vorteilhaft herausgestellt, dass die im vorliegenden Zusammenhang beschriebene Guanidinoessigsäure und deren Salze im Gegensatz zum Kreatin somit tatsächlich erst nach der Resorption, vor allem in der Leber, in Kreatin umgewandelt werden. Somit wird der überwiegende Teil der eingesetzten Verbindungen, im Gegensatz zum bekannten Kreatin, nicht bereits im Vorfeld durch Instabilitätsreaktionen abgebaut und ausgeschieden, sondern tatsächlich den physiologischen Anwendungsbereichen zur Verfügung gestellt. Die Guanidinoessigsäure und deren Salze in Kombination mit Cholin und/oder Betain führen gemäß Erfindung, somit wiederum im Gegensatz zum Kreatin und dessen Derivaten, zu deutlich höheren Kreatingehalten in den Zielorganen. Eine gleichzeitige Einnahme von großen Mengen an Zuckern oder Proteinen ist
durch die vorliegende Erfindung nicht mehr notwendig und ist aus ernährungsphysiologischer Sicht von großem Vorteil, da im Körper die Auschüttung von unphysiologischen Mengen an Insulin vermieden wird. Weiterhin konnte gezeigt werden, dass Guanidinoessigsäure und ihre Salze in Kombination mit Cholin und/oder Betain unter Bedingungen, wie sie bei der industriellen Herstellung von Nahrungsmitteln und Futtermitteln auftreten, eine sehr hohe Stabilität besitzen und weiterhin in praktisch jeder Darreichungsform lagerstabil sind. Guanidinoessigsäure in Kombination mit Cholin und/oder Betain zeigt hierbei klare Vorteile gegenüber Kreatin. Die Vorteile der mit der Erfindung beanspruchten Verwendung waren in ihrer Gesamtheit so nicht vorherzusehen.

Als Anwendungsbereiche bei Menschen und Wirbeltieren beansprucht die vorliegende Erfindung den Einsatz von Guanidinoessigsäure in Kombination mit Cholin und/oder Betain zur Verbesserung der Gehirnleistung durch orale Supplementierung mit Guanidinoessigsäure und deren Salze in Kombination mit Cholin und/oder Betain. Hierbei wurden sowohl das Langzeitgedächtnis als auch das Kurzzeitgedächtnis positiv beeinflusst. Auch konnten sich die Probanden deutlich länger konzentrieren.

Auch das Knochenwachstum und die Mineralisierung der Knochen sowie das Knorpelwachstum konnten durch Guanidinoessigsäure bzw. deren Salze in Kombination mit Cholin und/oder Betain verbessert werden. Hierbei zeigte sich, dass das erfindungsgemäße Mittel im Vergleich zu Kreatin zu einer noch besseren Einlagerung von Calcium in den Knochen führt. Weiterhin konnten antioxidative und neuroprotektive Effekte beobachtet werden.

Guanidinoessigsäure in Kombination mit Cholin und/oder Betain eignet sich auch zur Milderung von Alterungsprozessen, wobei die Muskelmasse, die Menge an Gewebswasser, die Calciumwerte in der Zelle und die Zellalterung positiv beeinflusst wurden.

Ein weiterer Anwendungsbereich von Guanidinoessigsäure in Kombination mit Cholin und/oder Betain ist die Vorbeugung von Entzündungsprozessen und die Stärkung des Immunsystems. In einem Experiment wurden Ratten mit Guanidinoessigsäure und Cholinchlorid, Kreatin Monohydrat oder einem Placebo gefüttert und nach 6 Wochen einer bakteriellen Infektion (Staphylococcus aureus) ausgesetzt. Die Supplementierung mit Guanidinoessigsäure und Cholinchlorid führte zu einer deutlich höheren Überlebensrate im Vergleich zu der Kreatin- und auch zu der KontrollGruppe. Weiterhin löste die Infektion bei der Guanidinoessigsäure-Gruppe deutlich abgemilderte Entzündungsprozesse aus. Dieser Effekt konnte über die Messung des CRP-Proteins im Serum der Ratten belegt werden.

Guanidinoessigsäure und deren Salze in Kombination mit Cholin und/oder Betain eignen sich auch in hervorragender Weise zur Senkung des Cholesterin- und Triglyceridwertes sowie zur Senkung des Blutzuckerspiegels, wobei die beobachteten Effekte in allen Fällen die Wirkung von Kreatin überstiegen.

Aus den für den erfindungsgemäßen Verwendungszweck in Frage kommenden Guanidinoessigsäure-Salzen haben sich insbesondere Salze als günstig erwiesen, die mit Asparaginsäure, Ascorbinsäure, Brenztraubensäure, Bernsteinsäure, Fumarsäure, Gluconsäure, α-Ketoglutansäure, Oxalsäure, Pyroglutaminsäure, 3-Nicotinsäure, Milchsäure, Zitronensäure, Maleinsäure, Schwefelsäure, Essigsäure, Ameisensäure, Salzsäure und Phosphorsäure, 2-Hydroxybenzoesäure, L-Carnitin, Acetyl-L-Carnitin, Taurin, Betain, Cholin, Methionin und Liponsäure erhalten werden, wobei Kalium-, Calcium- oder Natriumguanidinoacetat besonders geeignet sind. Es können natürlich auch Mischungen aus Guanidinoessigsäure mit einem oder mehreren der oben genannten Salze oder Mischungen aus den oben genannten Salzen eingesetzt werden.

Gemäß einer bevorzugten Ausführungsform wird das erfindungsgemäße Mittel in Kombination mit weiteren Methylgruppen-Donoren, ausgewählt aus der Gruppe Dimethylglycin, Sarkosin, Folsäure und Methionin, oder eine Mischung dieser Komponenten, eingesetzt.

Als weiterer Vorteil der Verwendung gemäß Erfindung hat sich herausgestellt, dass die Guanidinoessigsäure und deren Salze in einem relativ breiten Dosierbereich eingesetzt werden können. Dabei sind sowohl die Einzeldosen als auch die Tagesdosen keinen Einschränkungen unterworfen. Im Hinblick auf die Verwendung als Nahrungsergänzungsmittel, medizinische Zubereitung, Funktionsnahrungsmittel und Futtermittel kommen vorzugsweise Einzeldosen von 0,001 bis 0,3 g/kg in Frage, wobei Einzeldosen von 0,05 bis 0,15 g/kg als besonders bevorzugt anzusehen sind.

Auch die Methyl-Gruppen-Donoren Cholin und Betain können in einem relativ breiten Dosierbereich eingesetzt werden. Im Hinblick auf die Verwendung als Nahrungsergänzungsmittel, medizinische Zubereitung, Funktionsnahrungsmittel und Futtermittel kommen bei Cholin und Betain vorzugsweise Einzeldosen von 0,001 bis 0,4 g/kg in Frage, wobei Einzeldosen von 0,03 bis 0,25 g/kg als besonders bevorzugt anzusehen sind.

Das molare Verhältnis der eingesetzten Menge an Guanidinoessigsäure zu Cholin und/oder Betain liegt im Bereich von 1 : 1.

Es gilt als erfindungswesentlich, dass die beanspruchte Verwendung der Guanidinoessigsäure und deren Salze in Kombination mit Cholin und/oder Betain für Menschen oder Wirbeltiere, vorzugsweise Haustiere, Zucht- und Masttiere vorgenommen wird. Als Darreichungsformen kommen vor allem Pulver, Granulate, Pastillen, Kapseln, Pellets, Lösungen, Säfte oder Gelee-Produkte in Frage.
Als weiteren Anwendungsbereich sieht die vorliegende Erfindung den Einsatz als Futtermittel vor, wobei sich insbesondere Trocken-, Halbfeucht- und Nassfutter in der Form von Dosenfutter, Pellets, Biskuits, Kroketten, Nuggets, Flocken und Snacks eignen.

Als weiterer Aspekt der vorliegenden Erfindung wird die Guanidinoessigsäure und/oder deren Salze in Kombination mit Cholin und/oder Betain in Form einer essbaren Matrix eingesetzt. Besonders vorteilhaft ist es hierbei, wenn die Guanidinoessigsäure oder deren Salze als kristalline oder pulverförmige Feststoffe in der jeweiligen essbaren Matrix vorliegen. In diesem Zusammenhang ist es als bevorzugt anzusehen, wenn durch die physikalischen Eigenschaften der essbaren Matrix eine Separation der Komponenten verzögert oder vorzugsweise verhindert wird. Diese bevorzugten Eigenschaften der essbaren Matrix können durch den Zusatz viskositätserhöhender Substanzen, wie etwa Alginate, Xanthan, Guarkernmehl oder Johannisbrotkernmehl, unterstützt werden. Als essbare Matrices eignen sich somit feste, halbflüssige und flüssige Nahrungsmittel. Zur Verbesserung des Geschmacks kann es von Vorteil sein, Guanidinoessigsäure oder deren Salze für diesen Einsatzzweck zu vermahlen. Bevorzugt wird die einzuarbeitende Substanz homogen in der essbaren Matrix verteilt, wobei dies manuell (z.B. vom Endverbraucher) und/oder mechanisch möglich ist. Beispielhaft seien hier Molkereiprodukte, wie etwa Joghurt, Molke, Käse und Milch genannt. Weiterhin eignet sich die Guanidinoessigsäure zur Einarbeitung in industriell hergestellte Fertig- und Halbfertigprodukte wie etwa Nudeln, Müsli, Cerialien, Backwaren, Fertiggerichte, Riegel, Brot, Wurst und Getränke, in welche die Guanidinoessigsäure(-Salze) während des Herstellungsprozesses eingearbeitet wurde(n). Hierbei hat sich überraschend herausgestellt, dass durch diese Einarbeitung die Stabilität der Guanidinoessigsäure oder deren Salze noch weiter erhöht werden kann, wobei der pH-Wert der essbaren Matrix keinen wesentlichen Einfluss auf die Stabilität ausübt. Somit sind praktisch alle Nahrungsmittel in der erfindungsgemäßen Weise einsetzbar, wobei ein pH-Wert der Matrix zwischen 2 und 11 besonders geeignet ist. Weiterhin stellen die vorgeschlagenen Nahrungsmittelerzeugnisse eine sehr bequeme und zweckmäßige Form für die tägliche Aufnahme von Guanidinoessigsäure und deren Salze in Kombination mit Cholin und/oder Betain dar.

Die erfindungsgemäßen Formulierungen enthalten nach 90 Tagen Lagerung bei Raumtemperatur noch mindestens 90% der ursprünglich eingesetzten Guanidinoessigsäure und/oder deren Salze. In den meisten Matrices liegt die noch vorhandene Menge nach 90 Tagen bei über 95%, insbesondere bei über 99%. Diese hohe Stabilität ist für Kreatin unter vergleichbaren Bedingungen bisher nicht erreicht worden, wobei insbesondere auf die Druckschrift EP 1180944 verwiesen wird.

Sowohl Cholin als auch Betain werden in einer bevorzugten Ausführungsform gleichzeitig mit Guanidinoessigsäure in die essbaren Matrices eingearbeitet und sind sowohl unter den Bedingungen der Herstellung als auch der Lagerung der essbaren Matrices vollständig stabil.

In Abhängigkeit vom jeweiligen konkreten Verwendungsfall kann es durchaus empfehlenswert sein, das erfindungsgemäß vorgeschlagene Mittel in Kombination mit anderen physiologisch aktiven Nährstoffen der Reihe Kohlenhydrate, Fette, Aminosäuren, Proteine, Vitamine, Mineralstoffe, Spurenelemente, Koffein, Taurin und deren Derivate und Mischungen daraus einzusetzen.

Insgesamt bietet die vorliegende Erfindung mit den neuen Einsatzgebieten für Guanidinoessigsäure in Kombination mit Cholin und/oder Betain weit mehr als nur neue Alternativen zu den bekannten Kreatin-Verbindungen an, da die erfindungsgemäße Zusammensetzung die Nachteile von Kreatin überwindet und durch ihre bessere Bioverfügbarkeit und höhere Wirksamkeit eine sehr deutliche Verbesserung darstellt.

Die nachfolgenden Beispiele verdeutlichen die Breite der vorliegenden Erfindung.

### Beispiele

### Beispiel 1

### Nahrungsergänzungsmittel und medizinische Zubereitungen

Nachfolgend sind typische Zusammensetzungen von wohlschmeckenden Formulierungen aufgeführt, deren Bestandteile bei Raumtemperatur trocken abgemischt worden sind. Es wird empfohlen, die pulverförmigen Formulierungen vor deren oraler Aufnahme in 200 ml Fruchtsaft und/oder Wasser zu lösen.

| | | |
|---|---|---|
| 1.1 | 1.500 mg | Glucosamin |
| | 750 mg | Guanidinoessigsäure |
| | 720 mg | Magnesium-L-hydrogenaspartat |
| | 500 mg | Ascorbinsäure |
| | 720 mg | Cholincitrat |
| | | |
| 1.2 | 400 mg | Chondroitinsulfat |
| | 500 mg | Guanidinoessigsäurepyruvat |
| | 2.000 mg | Dicalciumphosphat |
| | 400 mg | (MgCO₃)₄·Mg(OH)₂·5H₂O = ca. 100 Mg |
| | 500 mg | Vitamin C |
| | 800 mg | Betain |
| | | |
| 1.3 | 1.000 mg | Glucosamin |
| | 300 mg | Cholinchlorid |
| | 2.800 mg | Guanidinoessigsäure |
| | 3.100 mg | Creatinol-O-phosphat |

### Beispiel 2

### Futtermittelzusatz

2.1. Eine Formulierung bestehend aus 5.000 mg Guanidinoessigsäure, 3000 mg Cholinchlorid und 5.000 mg Inulin wurde in eine typische Rezeptur für Futterpellets zur Futterergänzung von Hunden eingebracht.
2.2 Eine Formulierung bestehend aus 7.000 mg Guanidinoessigsäurelipoat, 5000 mg Cholinchlorid, 750 mg Carnitintatrat, 100 mg Succrosestearat, 160 mg Talkum und 1.090 mg Fructose wurde in die Basismasse für Hundebisquits eingebracht.
2.3 Als Masterbatch wurde in eine handelsübliche Katzendosenfuttermischung homogen folgende Formulierung eingebracht: 3.000 mg Guanidinoessigsäurepyruvat, 4000 mg Cholinchlorid, 3.000 mg Kreatin, 40 mg Magnesiumstearat, 25 mg Carboxymethylcellulose und 135 mg Lactose.

### Beispiel 3

### Funktionsnahrungsmittel

3.1 Eine Mischung aus 5 kg Guanidinoessigsäure und 8 kg Cholinchlorid wird in 900 kg einer Brotbackmischung homogen eingearbeitet.
3.2 Eine Mischung aus 5 kg Betain und 7 kg Guanidinoessigsäurelipoat wird homogen in eine Streichkäsezubereitung eingearbeitet.
3.3 In eine Fruchtzubereitung für ein Joghurt werden 20 kg Betain, 10 g Folsäure und 14 kg Guanidinoessigsäure vor der Sterilisierung homogen eingearbeitet.
3.4 In einen Müsliriegel werden 3 g Guanidinoessigsäure und 3 g Betain homogen eingebracht.
3.5 Conflakes werden aus einer Mischung aus Mais, Malzextrakt, Salz, 30 kg Guanidinoessigsäure und 40 kg Cholinbitatrat hergestellt.

### Beispiel 4

### Biologische Verfüdbarkeit und Wirkung

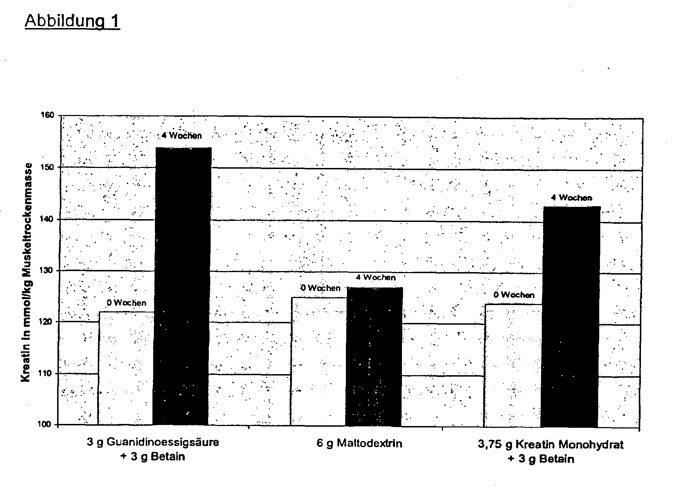

4.1 Vier Probanden-Gruppen von jeweils zwanzig Personen wurden so zusammengestellt, dass in allen Gruppen etwa der gleiche durchschnittliche Ausgangswert von Kreatin in der Muskeltrockenmasse vorhanden war.
Über vier Wochen wurde einer Gruppe täglich ein erfindungsgemäßes Funktionsnahrungsmittel (Müsliriegel) nach Beispiel 3.4 verabreicht, welches 3 g Guanidinoessigsäure und 3 g Betain enthielt (Gruppe1). Die zweite Gruppe erhielt einen Müsliriegel mit 6 g Maltodextrin (Gruppe 2) und die dritte Gruppe einen Müsliriegel welcher 3,75 g Kreatin Monohydrat (äquimolar zu 3 g Guanidinoessigsäure) und 3 g Betain enthielt (Gruppe 3). Die vierte Gruppe erhielt einen Müsliriegel mit 3 g Guanidinoessigsäure (Gruppe 4). Unmittelbar vor der Studie und nach vier Wochen der Einnahme wurden die Kreatin-Gehalte im Muskel mittels Muskelbiopsie gemessen. Die Ergebnisse sind in der Abbildung 1 dargestellt.
Während der 4 Wochen wurde mit allen Probanden täglich ein 60-minütiges standadisiertes Fitnessprogramm durchgeführt. Die körperliche und geistige Leistungsfähigkeit sowie eine Reihe von Blutparametern wurden vor und nach der vierwöchigen Einnahme gemessen. Die Ergebnisse sind in Tabelle 1 zusammengefasst.

**Tabelle 1**

| | Cholesterinwert | Triglyceridwert | BDS (1) | RAPM (2) | Knochendichte | Cystinwert | Blutzuckerspiegel |
|---|---|---|---|---|---|---|---|
| Gruppe 1 | -8% | -26% | 2,7 | 5,7 | 4% | -24% | -8% |
| | | | | | | | |
| Gruppe 2 | 1% | -3% | 0,8 | 1,5 | 0% | -3% | 2% |
| | | | | | | | |
| Gruppe 3 | -5% | -21% | 2,1 | 4,4 | 2,50% | -15% | -5% |
| | | | | | | | |
| Gruppe 4 | -4% | -22% | 2,3 | 4,6 | 2,80% | -14% | -5% |

Tabelle 1 stellt die Differenz zwischen der jeweiligen Ausgangsmessung und der Messung nach der vierwöchigen Supplementation dar.
(1) BDS (Backward Digit Span): Den Probanden wurde eine Zahlenreihe vorgelesen, welche diese rückwärts wiederholen mussten. Die Anzahl der korrekt wiederholten Zahlen vor der Supplementation wurde von der Anzahl der korrekt wiederholten Zahlen nach der Supplementation subtrahiert.
(2) RAPM (Raven's Advanced Progressive Matrices): Mit den Probanden wurde ein standadisierter Intelligenztest durchführt. Die erzielte Punktzahl vor der Supplementation wurde von der Punktzahl nach der Supplementation subtrahiert.

4.2 Drei Gruppen F344-Ratten (Charles River Wiga GmbH, Sulzfeld) mit jeweils 25 Tieren wurden für 6 Wochen mit einem Standardfutter gefüttert, welchem
(Gruppe 1) Guanidinoessigsäure (0,5%) und Cholinchlorid (0,5%),
(Gruppe 2) Kreatin Monohydrat (0,63%) und Cholinchlorid (0,5%) und
(Gruppe 3) Maltodextrin (1%) zugegeben wurde.
Allen Tieren wurden nach den 6 Wochen 1,0 ml einer Staphylococcus aureus Suspension (3X10⁶ Bakterien pro ml) intravenös injiziert.
Die Überlebensrate und der CRP-Serumspiegel der überlebenden Tiere wurde nach 3 Tagen beurteilt (Tabelle 2).

**Tabelle 2**

| | Überlebensrate | CRP-Serumspiegel |
|---|---|---|
| Gruppe 1 | 80% | 3,4 mg/dl |
| | | |
| Gruppe 2 | 68% | 4,5 mg/dl |
| | | |
| Gruppe 3 | 48% | 7,3 mg/dl |

| | | |
|---|---|---|
| (CRP= C-reaktives Protein) | | |

## Patentansprüche

1. Verwendung von Guanidinoessigsäure und/oder deren Salzen in Kombination mit Cholin und/oder Betain zur Herstellung eines Mittels zur Verbesserung der Gehirnleistung bei Menschen oder Wirbeltieren, wobei das molare Verhältnis der eingesetzten Menge an Guanidinoessigsäure zu Cholin und/oder Betain im Bereich von 1 : 1 liegt.

2. Verwendung von Guanidinoessigsäure und/oder deren Salzen in Kombination mit Cholin und/oder Betain zur Herstellung eines Mittels zur Verbesserung des Knochenwachstums und der Mineralisierung der Knochen, zur Verbesserung des Knorpelwachstums bei Menschen oder Wirbeltieren, wobei das molare Verhältnis der eingesetzten Menge an Guanidinoessigsäure zu Cholin und/oder Betain im Bereich von 1 : 1 liegt.

3. Verwendung nach Anspruch 1 oder 2,
**dadurch gekennzeichnet,**
**dass** es sich bei Wirbeltieren um Haustiere, Zucht- und/oder Masttiere handelt.

4. Verwendung nach einem der Ansprüche 1 bis 3,
**dadurch gekennzeichnet,**
**dass** das Mittel Pulver, Granulate, Pastillen, Kapseln, Pellets, Lösungen, Säfte oder Gelee-Produkte umfasst.

5. Verwendung nach einem der Ansprüche 1 bis 3,
**dadurch gekennzeichnet,**
**dass** das Mittel eine essbare Matrix umfasst, in welche die Guanidinoessigsäure und/oder deren Salze in Kombination mit Cholin und/oder Betain eingearbeitet wurde(n).

6. Verwendung nach Anspruch 5,
**dadurch gekennzeichnet,**
**dass** es sich bei der essbaren Matrix um ein industriell hergestelltes Lebensmittel in Form eines Fertig- oder Halbfertigproduktes, Getränks oder Futtermittels handelt, in welches Guanidinoessigsäure und/oder deren Salze in Kombination mit Cholin und/oder Betain im Herstellungsprozess durch Mischen oder Lösen eingearbeitet wurde(n).

7. Verwendung nach einem der Ansprüche 1 bis 6,
**dadurch gekennzeichnet,**
**dass** das Mittel weitere physiologisch aktive Nährstoffe umfasst, die ausgewählt sind aus Kohlenhydraten, Fetten, Aminosäuren, Proteinen, Vitaminen, Mineralstoffen, Spurenelementen, Koffein, Taurin und deren Derivaten und Mischungen davon.

8. Verwendung nach einem der Ansprüche 1 bis 7,
**dadurch gekennzeichnet,**
**dass** es sich bei den Salzen der Guanidinoessigsäure um solche mit Äpfelsäure, Asparaginsäure, Ascorbinsäure, Bernsteinsäure, Brenztraubensäure, Fumarsäure, Glukonsäure, α-Ketoglutarsäure, Oxalsäure, Pyroglutaminsäure, 3-Nicotinsäure, Milchsäure, Zitronensäure, Maleinsäure, Schwefelsäure, Essigsäure, Ameisensäure, Salzsäure, Phosphorsäure, 2-Hydroxybenzoesäure, L-Carnitin, Acetyl-L-Carnitin, Taurin, Betain, Cholin, Methionin und/oder Liponsäure sowie Natrium, Kalium oder Calcium handelt oder/und dass die Guanidinoessigsäure(-Salze) mit weiteren Methyl-Gruppen-Donoren der Reihe Dimethylglycin, Sarkosin, Folsäure und Methionin oder einer Mischung dieser Komponenten kombiniert werden.

9. Verwendung nach einem der Ansprüche 1 bis 8,
**dadurch gekennzeichnet,**
**dass** die Guanidinoessigsäure und/oder deren Salze in Einzeldosen von 0,001 bis 0,3 g/kg Körpergewicht verabreicht wird.

10. Zusammensetzung für die menschliche Ernährung umfassend Guanidinoessigsäure in Kombination mit Cholin und/oder Betain, wobei die Guanidinoessigsäure als Feststoff in einer flüssigen, halbfesten oder festen essbaren Matrix verteilt ist,
**dadurch gekennzeichnet,**
**dass** das molare Verhältnis von Guanidinoessigsäure zu Cholin und/oder Betain bei etwa 1 : 1 liegt.

11. Zusammensetzung nach Anspruch 10,
**dadurch gekennzeichnet,**
**dass** es sich um Molkereiprodukte wie etwa Joghurt, Molke, Käse und Milch und industriell hergestellte Fertig- und Halbfertigprodukte wie etwa Nudeln, Müsli, Cerialien, Backwaren, Fertiggerichte, Riegel, Brot, Wurst und Getränke handelt.

12. Zusammensetzung nach einem der Ansprüche 10 oder 11,
**dadurch gekennzeichnet,**
**dass** der pH-Wert der Matrix zwischen 2 und 11 liegt.

13. Zusammensetzung nach einem der Ansprüche 10 bis 12,
**dadurch gekennzeichnet,**
**dass** diese nach 90 Tagen Lagerung bei Raumtemperatur noch mindestens 90 % der ursprünglich eingesetzten Menge an Guanidinoessigsäure und/oder deren Salze enthält.

14. Zusammensetzung nach einem der Ansprüche 10 bis 13,
**dadurch gekennzeichnet,**
**dass** die essbare Matrix viskositätserhöhende Substanzen, wie etwa Alginate, Xanthan, Guarkernmehl oder Johannisbrotkernmehl, umfasst.

## Claims

1. Use of guanidinoacetic acid and/or salts thereof, in combination with choline and/or betaine, for producing an agent for improving brain function in humans or vertebrates, wherein the molar ratio of the amount of guanidinoacetic acid used to choline and/or betaine is in the range of 1:1.

2. Use of guanidinoacetic acid and/or salts thereof, in combination with choline and/or betaine, for producing an agent for improving bone growth and the mineralisation of bones, for improving the cartilage growth in humans or vertebrates, wherein the molar ratio of the amount of guanidinoacetic acid used to choline and/or betaine is in the range of 1:1.

3. Use according to either claim 1 or claim 2, **characterised in that** vertebrates are pets, breeding animals and/or animals for fattening.

4. Use according to any of claims 1 to 3, **characterised in that** the agent includes powder, granulate, lozenges, capsules, pellets, solutions, juices or jelly products.

5. Use according to any of claims 1 to 3, **characterised in that** the agent comprises an edible matrix into which the guanidinoacetic acid and/or salts thereof has/have been incorporated in combination with choline and/or betaine.

6. Use according to claim 5, **characterised in that** the edible matrix is an industrially produced food in the form of a finished product or semi-finished product, drink or feed, into which guanidinoacetic acid and/or salts thereof has/have been incorporated, in combination with choline and/or betaine, in the production process by mixing or dissolving.

7. Use according to any of claims 1 to 6, **characterised in that** the agent comprises additional physiologically active nutrients selected from the group consisting of carbohydrates, fats, amino acids, proteins, vitamins, mineral substances, trace elements, caffeine, taurine, and derivatives and mixtures thereof.

8. Use according to any of claims 1 to 7, **characterised in that** the guanidinoacetic acid salts are those with malic acid, aspartic acid, ascorbic acid, succinic acid, pyruvic acid, fumaric acid, gluconic acid, α-ketoglutaric acid, oxalic acid, pyroglutamic acid, 3-nicotinic acid, lactic acid, citric acid, maleic acid, sulfuric acid, acetic acid, formic acid, hydrochloric acid, phosphoric acid, 2-hydroxybenzoic acid, L-carnitine, acetyl-L-carnitine, taurine, betaine, choline, methionine and/or lipoic acid, and sodium, potassium or calcium, and/or that the guanidinoacetic acid (salts) are combined with additional methyl group donors selected from the group consisting of dimethylglycine, sarcosine, folic acid and methionine, or a mixture of these components.

9. Use according to any of claims 1 to 8, **characterised in that** the guanidinoacetic acid and/or salts thereof are administered in single doses of from 0.001 to 0.3 g/kg body weight.

10. Composition for human nutrition, comprising guanidinoacetic acid in combination with choline and/or betaine, the guanidinoacetic acid being distributed as a solid in a liquid, semisolid or solid edible matrix, **characterised in that** the molar ratio of the guanidinoacetic acid to choline and/or betaine is approximately 1:1.

11. Composition according to claim 10, **characterised in that** it relates to dairy products such as yoghurt, whey, cheese, and milk, and industrially produced finished or semi-finished products such as pasta, muesli, cereals, baked products, ready meals, bars, bread, sausage, and drinks.

12. Composition according to either claim 10 or claim 11, **characterised in that** the pH of the matrix is between 2 and 11.

13. Composition according to any of claims 10 to 12, **characterised in that** said composition still contains at least 90 % of the originally used amount of guanidinoacetic acid or guanidinoacetic and/or salts thereof after 90 days of storage at room temperature.

14. Composition according to any of claims 10 to 13, **characterised in that** the edible matrix comprises viscosity-increasing substances such as alginate, xanthan, guar gum, or locust bean gum.

## Revendications

1. Utilisation d'acide guanidino-acétique et/ou de ses sels en combinaison avec de la choline et/ou de la bétaïne pour la fabrication d'un agent destiné à l'amélioration des performances cérébrales chez les humains ou les animaux vertébrés, dans laquelle le rapport molaire de la quantité utilisée d'acide guanidino-acétique à la choline et/ou à la bétaïne se situe dans la plage de 1 : 1.

2. Utilisation d'acide guanidino-acétique et/ou de ses sels en combinaison avec de la choline et/ou de la bétaïne pour la fabrication d'un agent destiné à l'amélioration de la croissance osseuse et de la minéralisation des os, pour l'amélioration de la croissance du cartilage chez les humains ou les animaux vertébrés, dans laquelle le rapport molaire de la quantité utilisée d'acide guanidino-acétique à la choline et/ou à la bétaïne se situe dans la plage de 1 : 1.

3. Utilisation selon la revendication 1 ou 2,
**caractérisée en ce que**
les animaux vertébrés sont des animaux domestiques, des animaux d'élevage et/ou des animaux destinés à l'engraissement.

4. Utilisation selon l'une des revendications 1 à 3,
**caractérisée en ce que**
l'agent comprend des poudres, granulés, pastilles, capsules, comprimés, solutions, jus ou des produits en gel.

5. Utilisation selon l'une des revendications 1 à 3,
**caractérisée en ce que**
l'agent comprend une matrice comestible dans laquelle l'acide guanidino-acétique et/ou ses sels a/ont été incorporé(s) en combinaison avec de la choline et/ou de la bétaïne.

6. Utilisation selon la revendication 5,
**caractérisée en ce que**
la matrice comestible est un aliment produit industriellement sous la forme d'un produit fini ou semi-fini, d'une boisson ou d'un aliment pour animal dans lequel l'acide guanidino-acétique et/ou ses sels a/ont été incorporé(s) en combinaison avec de la choline et/ou de la bétaïne dans le procédé de production par mélange ou dissolution.

7. Utilisation selon l'une des revendications 1 à 6,
**caractérisée en ce que**
l'agent comprend d'autres nutriments physiologiquement actifs qui sont choisis parmi les glucides, les lipides, les acides aminés, les protéines, les vitamines, les substances minérales, les oligo-éléments, la caféine, la taurine et leurs dérivés et mélanges.

8. Utilisation selon l'une des revendications 1 à 7,
**caractérisée en ce que**
les sels de l'acide guanidino-acétique sont des sels avec de l'acide malique, de l'acide aspartique, de l'acide ascorbique, de l'acide succinique, de l'acide pyruvique, de l'acide fumarique, de l'acide gluconique, de l'acide α-cétoglutarique, de l'acide oxalique, de l'acide pyroglutamique, de l'acide 3-nicotinique, de l'acide lactique, de l'acide citrique, de l'acide maléique, de l'acide sulfurique, de l'acide acétique, de l'acide formique, de l'acide chlorhydrique, de l'acide phosphorique, de l'acide 2-hydroxybenzoïque, de la L-cartinine, de l'acétyl-L-carnitine, de la taurine, de la bétaïne, de la choline, de la méthionine et/ou de l'acide lipoïque et du sodium, du potassium ou du calcium et/ou **en ce que** l'acide guanidino-acétique (ou ses sels) est/sont combiné(s) avec d'autres donneurs de groupes méthyle de la série de la diméthylglycine, de la sarcosine, de l'acide folique et de la méthionine ou d'un mélange de ces composants.

9. Utilisation selon l'une des revendications 1 à 8,
**caractérisée en ce que**
l'acide guanidino-acétique et/ou ses sels est/sont administré(s) en doses uniques de 0,001 à 0,3 g/kg de poids corporel.

10. Composition pour l'alimentation humaine comprenant de l'acide guanidino-acétique en combinaison avec de la choline et/ou de la bétaïne, dans laquelle l'acide guanidino-acétique est réparti sous forme solide dans une matrice comestible liquide, semi-solide ou solide,
**caractérisée en ce que**
le rapport molaire de l'acide guaninio-acétique à la choline et/ou à la bétaïne est d'environ 1 : 1.

11. Composition selon la revendication 10,
**caractérisée en ce**
**qu'**il s'agit de produits laitiers tels que yaourt, petit lait, fromage et lait et de produits finis et semi-finis produits industriellement tels que des pâtes, du muesli, des céréales, des produits de boulangerie, des plats cuisinés, des barres énergétiques, du pain, des saucisses et des boissons.

12. Composition selon l'une des revendications 10 ou 11,
**caractérisée en ce que**
le pH de la matrice se situe entre 2 et 11.

13. Composition selon l'une des revendications 10 à 12,
**caractérisée en ce que**
celle-ci contient après 90 jours de stockage à température ambiante encore au moins 90 % de la quantité utilisée initialement d'acide guanidino-acétique et/ou leurs sels.

14. Composition selon l'une des revendications 10 à 13,
**caractérisée en ce que**
la matrice comestible comprend des substances augmentant la viscosité telles que les alginates, le xanthane, la farine de guar ou la farine de caroube.
